# EUROPEAN PATENT APPLICATION

(11) **EP 3 913 366 A1**
(43) Date of publication of application: **24.11.2021**
(21) Application number: 19910385.4
(22) Date of filing: 23.12.2019
(51) Int. Cl.: G01N 33/53

(54) **METHOD AND KIT FOR DETERMINING POSSIBILITY OF ONSET OF IGA NEPHROPATHY**

(30) Priority: 17.01.2019 JP 2019006412
(71) Applicant: National University Corporation Okayama University, Kita-ku Okayama-shi Okayama 700-8530 (JP)
(72) Inventor: WADA, Jun, Okayama-shi, Okayama 700-8530 (JP); MISE, Koki, Okayama-shi, Okayama 700-8530 (JP)
(74) Representative: Plasseraud IP
(86) International application number: PCT/JP2019/050237
(87) International publication number: WO 2020/149105

(57) **Abstract**

Provided is a more accurate method for determining the possibility that a subject has developed IgA nephropathy. A method of determining the possibility that a subject has developed IgA nephropathy, in accordance with an aspect of the present invention, includes the step of determining the level of at least one glycan in a sample taken from the subject, the at least one glycan being at least one glycan that binds to at least one lectin selected from the group consisting of ACA, MAH, ABA, STL, LEL, WGA, MPA, Jacalin, MAL_I, PNA, ACG, GSL_I_A4, ConA, SSA, AOL, and GSL_II.

## Description

### Technical Field

The present invention relates to a method of determining the possibility that a subject has developed IgA nephropathy and a kit for determining the possibility that a subject has developed IgA nephropathy.

### Background Art

At present, chronic kidney disease (CKD) is suffered by 13,300,000 people. This number means that one in eight adults in Japan suffers from CKD, and it would be no exaggeration to say that CKD is a folk disease. CKD includes various types of diseases. One of such diseases, chronic glomerulonephritis, is suffered by 6,466 people, which occupies 17.8% of all types of CKD. Out of the chronic glomerulonephritis of different types, the disease suffered by the largest number of patients is IgA nephropathy.

Therapeutic methods corresponding to such kidney diseases have been established, and therefore what is important is a diagnostic method to identify the type of kidney disease affecting the patient. That is, the following (i) and (ii) are important: (i) a diagnostic method involving identifying IgA nephropathy patient(s) from among unspecified human population; and (ii) a diagnostic method involving identifying IgA nephropathy patient(s) from among a population of chronic glomerulonephritis patients. Typically, the diagnosis of these kidney diseases is confirmed by renal biopsy. However, for cases which are high risk for renal biopsy, it is sometimes necessary to make a diagnosis based only on clinical information without carrying out renal biopsy. Furthermore, since renal biopsy is an invasive test, a diagnosis of kidney diseases itself has not been done in cases where, for example:
(i) the subject showed only a slight urinary finding and therefore the possibility of a kidney disease has been overlooked and (ii) renal biopsy has not been done because of a risk to the body.

Under such circumstances, methods of diagnosing IgA nephropathy not relying on renal biopsy have been studied and reported. For example, Non-patent Literature 1 reports that ELISA using a monoclonal antibody that binds to galactose-deficient abnormal IgA1 is better in robustness than a conventional assay using a lectin called helix aspersa agglutinin (HAA), as a method of diagnosing IgA nephropathy. Non-patent Literature 2 studies and reports a method of diagnosing IgA nephropathy using a combination of four clinical factors (hematuria, proteinuria, serum IgA level, and serum IgA/C3 ratio).

Other literatures that may relate to the present invention are, for example, Patent Literatures 1 to 4. The following briefly discusses a difference between each literature and the present invention.

Patent Literature 1 discloses a technique to diagnose diabetes, diabetic early nephropathy, and diabetic nephropathy. Patent Literature 2 discloses a technique to analyze the interaction between protein and glycan, and does not mention the diagnosis of a specific disease. Patent Literature 3 discloses a technique to determine the stage of diabetic nephropathy. Patent Literature 4 discloses a technique to determine the chance of future deterioration in renal function. The techniques disclosed in these literatures differ from the present invention at least in that they are not a method of diagnosing IgA nephropathy. Specifically, Patent Literatures 1 to 3 do not disclose IgA nephropathy, and Patent Literature 4 does not disclose "diagnosis at the point in time at which the test was carried out" (the technique disclosed in Patent Literature 4 is to predict future renal prognosis, and does not make a diagnosis at present).

Furthermore, none of Patent Literatures 1 to 4 disclose a kit (a kit including a combination of specific lectins) disclosed in the present specification.

### Citation List

### [Patent Literatures]

[Patent Literature 1] Japanese Patent Application Publication, Tokukaihei, No. 10-332690
[Patent Literature 2] Pamphlet of International Publication No. WO 2005/064333
[Patent Literature 3] Japanese Patent Application Publication, Tokukai, No. 2010-256132
[Patent Literature 4] Pamphlet of International Publication No. WO 2018/181292

### [Non-patent Literatures]

[Non-patent Literature 1] Yasutake J, Suzuki Y, Suzuki H, et al. Novel lectin-independent approach to detect galactose-deficient IgA1 in IgA nephropathy. Nephrol Dial Transplant. 2015;30:1315-1321.
[Non-patent Literature 2] Nakayama K, Ohsawa I, Maeda-Ohtani A, et al. Prediction of diagnosis of immunoglobulin A nephropathy prior to renal biopsy and correlation with urinary sediment findings and prognostic grading. J Clin Lab Anal. 2008;22(2):114-118.

### Summary of Invention

### Technical Problem

However, the foregoing conventional techniques still have some room for an improvement in accuracy of diagnosis of IgA nephropathy. Specifically, with regard to the diagnostic method disclosed in Non-patent Literature 1, the accuracy of diagnosis of IgA nephropathy has not actually been verified, and its usefulness is unknown. Furthermore, the diagnostic method disclosed in Non-patent Literature 2 is not good enough in terms of sensitivity and specificity, and there have been no reports on verification of the validity of this diagnostic method.

An object of an aspect of the present invention is to provide a more accurate method for determining the possibility that a subject has developed IgA nephropathy.

### Solution to Problem

The present invention includes the following features.

<1> A method of determining the possibility that a subject has developed IgA nephropathy, the method including the step of:
   determining a level of at least one glycan in a sample taken from the subject, the at least one glycan being at least one glycan that binds to at least one lectin selected from the group consisting of ACA, MAH, ABA, STL, LEL, WGA, MPA, Jacalin, MAL_I, PNA, ACG, GSL_I_A4, ConA, SSA, AOL, and GSL_II.
<2> A method of determining the possibility that a subject has developed IgA nephropathy, the method including the steps of:
   determining a level of at least one glycan in a first sample taken from the subject, the at least one glycan being at least one glycan that binds to at least one lectin selected from the group consisting of ACA, MAH, ABA, MPA, Jacalin, LEL, ACG, STL, GSL_I_A4, WGA, SSA, PNA, ConA, Calsepa, AOL, SNA, UDA, LCA, GSL_II, UEA_I, LTL, MAL_I, TJA_I, ECA, PWM, PSA, AAL, DSA, BPL, TJA_II, NPA, PHA_E, RCA120, EEL, SBA, HPA, GNA, HHL, PTL_I, TxLC_I, PHA_L, GSL_I_B4, DBA, WFA, and VVA; and
   measuring a biomarker in a second sample taken from the subject, the biomarker being other than the level of the at least one glycan.
<3> A method of determining the possibility that a subject having a primary glomerular disease or a subject suspected of having a primary glomerular disease has developed IgA nephropathy, the method including the step of:
   determining a level of at least one glycan in a sample taken from the subject, the at least one glycan being at least one glycan that binds to at least one lectin selected from the group consisting of ACA, MAH, ABA, MPA, Jacalin, LEL, ACG, STL, GSL_I_A4, WGA, SSA, PNA, ConA, Calsepa, AOL, SNA, UDA, LCA, GSL_II, UEA_I, LTL, MAL_I, TJA_I, ECA, PWM, PSA, AAL, DSA, BPL, TJA_II, NPA, PHA_E, RCA120, SBA, GNA, HHL, PTL_I, TxLC_I, PHA_L, DBA, WFA, and VVA.
<4> A method of determining the possibility that a subject having a primary glomerular disease or a subject suspected of having a primary glomerular disease has developed IgA nephropathy, the method including the steps of:
   determining a level of at least one glycan in a first sample taken from the subject, the at least one glycan being at least one glycan that binds to at least one lectin selected from the group consisting of ACA, MAH, ABA, MPA, Jacalin, LEL, ACG, STL, GSL_I_A4, WGA, SSA, PNA, ConA, Calsepa, AOL, SNA, UDA, LCA, GSL_II, UEA_I, LTL, MAL_I, TJA_I, ECA, PWM, PSA, AAL, DSA, BPL, TJA_II, NPA, PHA_E, RCA120, EEL, SBA, HPA, GNA, HHL, PTL_I, TxLC_I, PHA_L, GSL_I_B4, DBA, WFA, and VVA; and
   measuring a biomarker in a second sample taken from the subject, the biomarker being other than the level of the at least one glycan.
<5>
   (i) A method of determining the possibility that a subject has developed IgA nephropathy or (ii) a method of determining the possibility that a subject having a primary glomerular disease or a subject suspected of having a primary glomerular disease has developed IgA nephropathy,
      the method including the step of determining a level of at least one glycan in a sample taken from the subject, the at least one glycan being at least one selected from the group consisting of:
      (Galβ1-4GlcNAc)ₙ;
      (GlcNAcβ1-4)ₙ;
      (GlcNAcβ4MurNAc)ₙ;
      Agalactosylated tri/tetra antennary glycans;
      Fucα 1-2Galβ1-4GlcNAc;
      Fucα 1-6GlcNAc;
      GalNAc;
      Galβ 1-3GalNAc;
      GlcNAc;
      High-Man including Manα1-6(Manα 1-3)Man;
      Siaα2-3Galβ1-3(Siaα2-6)GalNAc;
      Siaα2-3Galβ1-4GlcNAc;
      Siaα2-6Gal/GalNAc; and
      α-GalNAc.
<6> The method described in <2> or <4>, in which the biomarker other than the level of the at least one glycan is at least one selected from occult hematuria, proteinuria, serum IgA, and serum IgA/C3 ratio.
<7> The method described in any one of <1> to <6>, in which the sample used in the step of determining the level of the at least one glycan is a urine sample.
<8>
   (i) A kit for determining the possibility that a subject has developed IgA nephropathy or (ii) a kit for determining the possibility that a subject having a primary glomerular disease or a subject suspected of having a primary glomerular disease has developed IgA nephropathy,
      the kit including at least one lectin selected from the following group A, at least one lectin selected from the following group B, and at least one lectin selected from the following group C, and not including other lectins:
      Group A: ACA, MAH, and ABA;
      Group B: GSL_I_A4; and
      Group C: GSL_II, MAL_I, AOL, PNA, SNA, and HPA.
<9>
   (i) A kit for determining the possibility that a subject has developed IgA nephropathy or (ii) a kit for determining the possibility that a subject having a primary glomerular disease or a subject suspected of having a primary glomerular disease has developed IgA nephropathy,
      the kit including lectins that bind to at least one glycan selected from the following group a, at least one glycan selected from the following group b, and at least one glycan selected from the following group c, and not including other lectins:
      Group a: Galβ1-3GalNAc and Siaα2-3Galβ1-3(Siaα2-6)GalNAc;
      Group b: α-GalNAc;
      Group c: Agalactosylated tri/tetra antennary glycans, Fucα1-2Galβ1-4GlcNAc, Fucα1-6GlcNAc, GlcNac, Galβ1-3GalNAc, Siaα2-3Galβ1-4GlcNAc, Siaα2-6Gal/GalNAc, and α-GalNAc.

### Advantageous Effects of Invention

An aspect of the present invention makes it possible to provide a more accurate method for determining the possibility that a subject has developed IgA nephropathy.

### Description of Embodiments

The following description will discuss examples of embodiments of the present invention in detail. The present invention is not, however, limited to the embodiments below. Any numerical range expressed as "A to B" in the present specification means "not less than A and not more than B", unless otherwise stated.

### [1. Level of glycan that binds to lectin]

In an aspect, a method in accordance the present invention includes the step of determining the level of glycan(s) that binds to specific lectin(s) in a sample taken from a subject.

### (Method of determining the level of glycan that binds to lectin)

The inventors of the present invention have found that it is possible to determine whether a subject has developed IgA nephropathy, on the basis of the results of an assay using lectin(s) of certain type(s) (that is, on the basis of lectin signal intensity) (such an assay is hereinafter referred to as "lectin assay"). It is generally known that a specific glycan specifically binds to specific lectin(s). Therefore, the inventors' finding can also be described as follows: "it is possible to determine whether a subject has developed IgA nephropathy on the basis of the level of glycan(s) that binds to certain lectin(s)".

Therefore, a method for carrying out a "step of determining the level of a glycan that binds to a lectin X" is not limited, provided that the step is a "step of determining the level of a glycan that has a specific structure that binds to a lectin X". That is, the "step of determining the level of a glycan that binds to a lectin X" is not limited to a lectin assay. Examples of methods other than the lectin assay include liquid chromatography, mass spectrometry, ELISA, two-dimensional electrophoresis, protein array, bead assay, flow cytometry, BioPlex (registered trademark), and the like.

Note, however, that the determination of the level of glycan(s) via a lectin assay is preferred, because a pretreatment of a sample is easy. Especially in cases where a urine sample is used as a sample, a lectin assay is more preferred because it eliminates the need for the processes of concentrating the urine sample, removing proteins (such as albumin and IgG) contained in large amounts in the sample, and the like.

In an embodiment, the act "determine the level of a glycan" can involve quantization of the amount of the glycan contained in a sample. A specific example of this is measuring the concentration of the glycan in the sample.

In another embodiment, the act "determine the level of a glycan" can be comparing the amount of the glycan contained in the sample with a predetermined reference value. A specific example of this is determining whether the concentration of the glycan in the sample is higher than a predetermined reference value (cutoff value) or lower than the predetermined reference value. In such a case, the predetermined reference value can be set as appropriate via a medical procedure, statistical procedure, or the like. A plurality of predetermined reference values may be set.

The relationship between the level of a glycan that binds to a lectin and the development of IgA nephropathy is determined via, for example, a statistical procedure (for example, see Examples of the present application). That is, for example, it is possible to determine whether "high glycan levels are related to the development of IgA nephropathy" or "low glycan levels are related to the development of IgA nephropathy", via a statistical procedure.

For example, for "one glycan" models (Model (1)) and/or "one glycan + clinical factors" models (Model (2)) of Examples of the present application, with regard to 45 lectins studied, low levels of glycans that bind to those lectins were found to be related to the development of IgA nephropathy.

For "two or more glycans + clinical factors" models (Model 3) of Examples of the present application, with regard to ACA, GSL_I_A4, MAH, and ABA, low levels of glycans that bind to those lectins were found to be related to the development of IgA nephropathy. In contrast, with regard to GSL_II, MAL_I, PNA, SNA, and AOL, high levels of glycans that bind to those lectins were found to be related to the development of IgA nephropathy.

As such, the relationship between the glycan levels and the development of IgA nephropathy can vary depending on the model employed. However, a person skilled in the art can decide the relationship between the glycan levels and the development of IgA nephropathy for each model, by carrying out routine work with reference to the descriptions in the present specification.

### (Subject)

In the present specification, the "subject" is not limited to a human. A method in accordance with an embodiment of the present invention can also be applied to non-human mammals. Examples of non-human mammals include even-toed ungulates (such as cattle, wild boars, pigs, sheep, and goats), odd-toed ungulates (such as horses), rodents (such as mice, rats, hamsters, and squirrels), lagomorphs (such as rabbits), carnivorous animals (such as dogs, cats, and ferrets), and the like. The non-human mammals not only include domestic animals and companion animals (pet animals) but also wild animals.

The present invention is to determine whether a subject has developed IgA nephropathy, on the basis of the level of glycan(s) in a sample. A glycan is less different in structure among species than protein and nucleic acid. Therefore, it is considered that the method in accordance with an embodiment of the present invention is effective enough for the foregoing non-human mammals.

### (Sample)

In the present specification, the "sample" is intended to mean anything taken from a subject, and not specifically limited to a particular one. The scope of the meaning of the term "sample" as used herein not only includes blood, cerebrospinal fluid, lymph fluid, breast milk, saliva, nasal discharge, sweat, urine, stool, expired air, and the like but also includes tissue lysate derived from a pathological specimen, live tissue lysate, cell lysate, and the like.

In an embodiment of the present invention, urine is used as the sample. A urine sample is preferred in that it is typically used as a biopsy specimen, that other indicators relating to renal functions can be examined concurrently, and that taking a urine sample is easy (especially in cases of human subjects), for example.

In an embodiment of the present invention, blood is used as the sample. A blood sample is preferred in that it is typically used as a biopsy specimen, that other indicators relating to renal functions can be examined concurrently, and taking a blood sample is easy (also in cases of non-human subjects), for example. The scope of the meaning of the term "blood" as used herein not only includes whole blood but also includes components of whole blood (such as serum, plasma, and clot).

### (Lectin)

The following are descriptions for lectins mentioned in embodiments which will be described later.
ACA: *Amaranthus caudatus* Agglutinin
MAH: *Maackia amurensis* Hemagglutitnin
ABA: *Amaranthus caudatus* Agglutinin
MPA: *Maclura pomifera* Agglutinin
Jacalin: Jackfruit Lectin
LEL: *Lycopersicon esculentum* Lectin
ACG: *Agrocybe cylindracea* Galectin
STL: *Solanum tuberosum* Lectin (potato lectin)
GSL_I_A4: *Griffonia simplicifolia* Lectin I A4
WGA: *Triticum vulgaris* Agglutinin
SSA: *Sambucus sieboldiana* lectin
PNA: Peanut Agglutinin
ConA: *Canavalia ensiformis* Agglutinin
Calsepa: *Calystegia sepiem* Lectin
AOL: *Aspergillus oryzae* Lectin
SNA: *Sambucus nigra* agglutinin
UDA: *Urtica dioica* Agglutinin
LCA: *Lens culinaris* Agglutinin
GSL_II: *Griffonia simplicifolia* Lectin II
UEA_I: *Ulex europaeus* Agglutinin I
LTL: *Lotus tetragonolobus* Lectin
MAL_I: *Maackia amurensis* Lectin I
TJA_I: *Trichosanthes japonica* agglutinin I lectin
ECA: *Erythrina cristagalli* Agglutinin
PWM: *Phytolacca americana* Agglutinin
PSA: *Pisum sativum* Agglutinin
AAL: *Aleuria aurantia* Lectin)
DSA: *Datura stramonium* Agglutinin
BPL: *Bauhinia purpurea* Lectin
TJA_II: *Trichosanthes japonica* Agglutinin-II)
NPA: *Narcissus pseudonarcissus* Agglutinin
PHA_E: *Phaseolus vulgaris* Erythroagglutinin
RCA120: *Ricinus communis* Agglutinin I
EEL: *Euonymus europaeus* Lectin
SBA: *Glycine max* Agglutinin
HPA: *Helix pomatia* Agglutinin
GNA: *Galanthus nivalis* Agglutinin
HHL: *Hippeastrum hybrid* Lectin
PTL_I: *Psophocarpus tetragonolobus* Lectin-I
TxLC_I: *Tulipa gesneriana* Lectin-I
PHA_L: *Phaseolus vulgaris* Leucoagglutin
GSL_I_B4: *Griffonia simplicifolia* Lectin I B4
DBA: *Dolichos biflorus* Agglutinin
WFA: *Wisteria floribunda* Agglutinin
VVA: *Vicia villosa* Lectin

These lectins are conventionally known lectins, and their amino acid sequence information are available from various databases. The relationship between the level of glycans that bind to these lectins and the development of IgA nephropathy has already been described earlier in the section "Method of determining the level of glycan that binds to lectin".

Note that, in cases where the levels of two or more glycans are to be determined in the method in accordance with an embodiment of the present invention, the two or more glycans preferably differ from each other in structure (this also applies to other embodiments described later). Specifically, in the method in accordance with an embodiment of the present invention, in cases where the level of a glycan x that binds to a lectin X and the level of a glycan y that binds to a lectin Y are to be determined, it is preferable that the glycan x and the glycan y differ from each other in structure. This is to avoid the issue of multicollinearity when preparing diagnostic models.

### [1-1. Determination with respect to unspecified human population]

An embodiment of the present invention is directed to a method of determining the possibility that a subject has developed IgA nephropathy, the method including the step of determining the level of at least one glycan in a sample taken from the subject, the at least one glycan being at least one glycan that binds to at least one lectin selected from the group consisting of ACA, MAH, ABA, STL, LEL, WGA, MPA, Jacalin, MAL_I, PNA, ACG, GSL_I_A4, ConA, SSA, AOL, and GSL_II.

The method in accordance with the present embodiment is to determine the possibility that a subject, belonging to an unspecified population, has developed IgA nephropathy. That is, the method in accordance with the present embodiment is to determine the possibility that a subject has developed IgA nephropathy in the circumstances in which it is unknown whether the subject has a kidney disease or not.

### [1-2. Determination with respect to population of subjects having primary glomerular disease]

Another embodiment of the present invention is directed to a method of determining the possibility that a subject having a primary glomerular disease or a subject suspected of having a primary glomerular disease has developed IgA nephropathy, the method including the step of determining the level of at least one glycan in a sample taken from the subject, the at least one glycan being at least one glycan that binds to at least one lectin selected from the group consisting of ACA, MAH, ABA, MPA, Jacalin, LEL, ACG, STL, GSL_I_A4, WGA, SSA, PNA, ConA, Calsepa, AOL, SNA, UDA, LCA, GSL_II, UEA_I, LTL, MAL_I, TJA_I, ECA, PWM, PSA, AAL, DSA, BPL, TJA_II, NPA, PHA_E, RCA120, SBA, GNA, HHL, PTL_I, TxLC_I, PHA_L, DBA, WFA, and VVA.

The method in accordance with the present embodiment is to determine the possibility that a subject, belonging to a population of subjects having (or suspected of having) a primary glomerular disease, has developed IgA nephropathy. That is, the method in accordance with the present embodiment is to determine the possibility that a subject has developed IgA nephropathy in the circumstances in which the subject has been found to have a primary renal disease (or the subject is suspected of having a primary renal disease). This method is carried out with respect to a more limited population than the diagnostic method described in the section [1-1].

A known medical technology can be used to know that "a subject has a primary glomerular disease" and "a subject is suspected of having a primary glomerular disease".

The method in accordance with the present embodiment need only be capable of determining the possibility that a subject has developed IgA nephropathy. However, in cases where it can be determined that a subject having a primary glomerular disease or a subject suspected of having a primary glomerular disease has not developed (unlikely to have developed) IgA nephropathy by use of the method in accordance with the present embodiment, it may be determined that the subject has developed (highly likely to have developed) a primary glomerular disease other than IgA nephropathy.

It is noted here that, in Japan, IgA nephropathy is usually categorized as a primary glomerular disease; however, according to the WHO classification, IgA nephropathy is categorized as a secondary glomerular disease. In the descriptions of the present specification, IgA nephropathy is regarded as a kind of primary glomerular disease in accordance with the classification in Japan.

In this regard, in cases of employing the WHO classification, the "method of determining the possibility that a subject having a primary glomerular disease or a subject suspected of having a primary glomerular disease has developed IgA nephropathy" can be rephrased as a "method of determining the possibility that a subject having a primary glomerular disease or IgA nephropathy or a subject suspected of having a primary glomerular disease or IgA nephropathy has developed IgA nephropathy". Even if the method is rephrased as such, the effect of the present embodiment, i.e., improving the accuracy of differentiation between IgA nephropathy and other primary glomerular diseases, can still be achieved.

### [2. Combination of level of glycan that binds to lectin with other biomarkers]

In a second aspect, a method in accordance with the present invention includes the steps of: (i) determining the level of at least one glycan in a first sample taken from a subject, the at least one glycan being at least one glycan that binds to at least one specific lectin; and measuring a biomarker in a second sample taken from the subject. In this method, the biomarker measured in the second sample differs from the at least one glycan which binds to at least one specific lectin and whose level is determined in the first sample.

The method in accordance with the present embodiment makes it possible to determine the possibility that a subject has developed IgA nephropathy with higher accuracy. In particular, when the level of glycan(s) is combined with biomarker(s) which has been conventionally used for diagnosis of IgA nephropathy, it is likely that the accuracy of diagnosis will further improve.

A method of determining the level of glycan(s) that binds to specific lectin(s), subject, sample, and lectin have already been described in the section [1].

### (Method for measuring biomarker in second sample)

A method for measuring a biomarker in a second sample can be selected as appropriate depending on the type of sample and the type of biomarker. The descriptions in the section [1] shall be referenced for the type of sample and the type of measurement method.

The second sample may be the same as or different from the first sample. In cases where a plurality of biomarkers are measured, the biomarkers may be measured from different second samples. For example, in Examples of the present application, the level of glycan(s) is measured from a first sample (urine), and serum IgA, occult hematuria, and proteinuria are measured from second samples (serum and urine).

A biomarker measured from a second sample may be a glycan. Specifically, the following configuration is also included in the present embodiment: the level of a glycan A that binds to a specific lectin is determined in a first sample; and the level of a glycan B that differs from the glycan A is determined in a second sample.

Examples of biomarker(s) measured from second sample(s) include age, gender, BMI, arterial pressure, HbA1c, estimated glomerular filtration rate (eGFR), the amount of urinary protein, occult hematuria, serum IgA, complement C3, serum IgA/C3 ratio, and the like. Out of those listed above, the amount of urinary protein, occult hematuria, serum IgA, and serum IgA/C3 ratio are regarded as biomarkers indicating the development of IgA nephropathy (see Non-patent Literature 2); therefore, when any of these is combined with glycan level, it is likely that an improved effect will be achieved.

### [2-1. Determination with respect to general population]

An embodiment of the present invention is directed to a method of determining the possibility that a subject has developed IgA nephropathy, the method including the steps of: determining the level of at least one glycan in a first sample taken from the subject, the at least one glycan being at least one glycan that binds to at least one lectin selected from the group consisting of ACA, MAH, ABA, MPA, Jacalin, LEL, ACG, STL, GSL_I_A4, WGA, SSA, PNA, ConA, Calsepa, AOL, SNA, UDA, LCA, GSL_II, UEA_I, LTL, MAL_I, TJA_I, ECA, PWM, PSA, AAL, DSA, BPL, TJA_II, NPA, PHA_E, RCA120, EEL, SBA, HPA, GNA, HHL, PTL_I, TxLC_I, PHA_L, GSL_I_B4, DBA, WFA, and VVA; and measuring a biomarker in a second sample taken from the subject, the biomarker being other than the level of the at least one glycan.

The method in accordance with the present embodiment is to determine the possibility that a subject belonging to an unspecified population has developed IgA nephropathy, similarly to the method described in the section [1-1].

In the step of determining the level of at least one glycan that binds to at least one lectin, the levels of two or more glycans may be determined. For example:
(1) the levels of glycans that bind to the lectins ACA, GSL_I_A4, and GSL_II, respectively, may be determined;
(2) the levels of glycans that bind to the lectins MAH, GSL_I_A4, and MAL_I, respectively, may be determined;
(3) the levels of glycans that bind to the lectins ABA, GSL_I_A4, and AOL, respectively, may be determined;
(4) the levels of glycans that bind to the lectins MAH, GSL_I_A4, and PNA, respectively, may be determined;
(5) the levels of glycans that bind to the lectins MAH, GSL_I_A4, and SNA, respectively, may be determined;
(6) the levels of glycans that bind to the lectins ABA, GSL_I_A4, and MAL_I, respectively, may be determined; and/or
(7) the levels of glycans that bind to the lectins ABA, GSL_I_A4, and GSL_II, respectively, may be determined.

The method in accordance with the present embodiment may be arranged such that, in the step of determining the level of at least one glycan that binds to at least one lectin, the levels of glycans including any of the above-stated combinations may be determined or the levels of glycans in any of the above-stated combinations alone may be determined. For example, (i) not only the levels of glycans that bind to the lectins ACA, GSL_I_A4, and GSL_II, respectively, but also the level of a glycan that binds to another lectin may be determined or (ii) the levels of glycans that bind to the lectins ACA, GSL_I_A4, and GSL_II, respectively, alone may be determined.

### [2-2. Determination with respect to population of subjects having primary glomerular disease]

Another embodiment of the present invention is directed to a method of determining the possibility that a subject having a primary glomerular disease or a subject suspected of having a primary glomerular disease has developed IgA nephropathy, the method including the steps of: determining the level of at least one glycan in a first sample taken from the subject, the at least one glycan being at least one glycan that binds to at least one lectin selected from the group consisting of ACA, MAH, ABA, MPA, Jacalin, LEL, ACG, STL, GSL_I_A4, WGA, SSA, PNA, ConA, Calsepa, AOL, SNA, UDA, LCA, GSL_II, UEA_I, LTL, MAL_I, TJA_I, ECA, PWM, PSA, AAL, DSA, BPL, TJA_II, NPA, PHA_E, RCA120, EEL, SBA, HPA, GNA, HHL, PTL_I, TxLC_I, PHA_L, GSL_I_B4, DBA, WFA, and VVA; and measuring a biomarker in a second sample taken from the subject, the biomarker being other than the level of the at least one glycan.

The method in accordance with the present embodiment is to determine the possibility that a subject, belonging to a population of subjects having (or suspected of having) a primary glomerular disease, has developed IgA nephropathy, similarly to the method described in the section [1-2]. Note that the descriptions in the section [1-2] are employed as descriptions for the present embodiment.

In the step of determining the level of at least one glycan that binds to at least one lectin, the levels of two or more glycans may be determined. For example, the levels of glycans that bind to the lectins MAH, GSL_I_A4, and HPA, respectively, may be determined.

The method in accordance with the present embodiment may be arranged such that, in the step of determining the level of at least one glycan that binds to at least one lectin, the levels of glycans including the above-stated combination may be determined or the levels of glycans in the above-stated combination alone may be determined. For example, (i) not only the levels of glycans that bind to the lectins MAH, GSL_I_A4, and HPA, respectively, but also the level of a glycan that binds to another lectin may be determined or (ii) the levels of glycans that bind to the lectins MAH, GSL_I_A4, and HPA, respectively, alone may be determined.

### [3. Determination of level of specific glycan]

As described earlier, lectins have the property that they each specifically bind to glycan(s) having specific structure(s). Therefore, in the present specification, "a glycan that binds to a lectin X" can also be specified by its structure. Table 1 below shows lectins mentioned in the present specification and examples of glycans that specifically bind to those lectins.

**[Table 1]**

| Lectin | Glycan that binds to lectin | Lectin | Glycan that binds to lectin |
|---|---|---|---|
| ACA | Galβ1-3GalNAc | ECA | Galβ4GlcNAc |
| MAH | Siaα2-3Galβ1-3(Siaα2-6)GalNAc | PWM | (GlcNAcβ4)ₙ |
| ABA | Galβ1-3GalNAc | PSA | Fucα6GIcNAc; High-Man |
| MPA | Galβ1-3GalNAc; GalNAc | AAL | Fucα6GlcNAc (core Fuc); Fucα3(Galβ4)GlcNAc (Lex) |
| Jacalin | Galβ1-3GalNAc; GalNAc | DSA | (GlcNAcβ4)n; triantennary/ tetraantennary N-glycans |
| LEL | (GlcNAcβ1-4)ₙ; (Galβ1-4GlcNAc)ₙ | BPL | Galβ1-3GalNAc; GalNAc |
| ACG | Siaα2-3Galβ1-4GlcNAc | TJA_II | Fucα2Galβ1; GalNAcβ1 |
| STL | (GlcNAcβ1-4)ₙ; (GlcNAcβ4MurNAc)ₙ | NPA | High-Man including Manα6Man |
| GSL_I_ A4 | α-GalNAc | PHA_E | N-glycans with outer Gal and bisecting GlcNAc |
| WGA | (GlcNAcβ1-4)ₙ | RCA120 | Galβ1-4GlcNAc |
| SSA | Siaα2-6Gal/ GalNAc | EEL | Galα1-3Galβ1-4GlcN Ac; Fucα2(Gala3)Galβ1-4 GlcNAc |
| PNA | Galβ1-3GalNAc | SBA | GalNAc; GalNAcα1-3Gal |
| ConA | High-Man including Manα1-6(Manα1-3)Man | HPA | α-GalNAc |
| Calsepa | High-Man (Man2-6); N-glycans including bisecting GlcNAc | GNA | High-Man including Manα3Man |
| AOL | Fucα1-6GlcNAc; Fucα1-2Galβ1-4GlcNAc | HHL | High-Man including Manα3Man or Manα6Man |
| SNA | Siaα2-6Gal/ GalNAc | PTL_I | α-GalNAc |
| UDA | GlcNAcβ4GlcNAc; Man5~Man9 | TxLC_I | Man3 core; bi- and tri-antennary N-glycans; GalNAc |

**[Table 1] (Continued)**

| | | | |
|---|---|---|---|
| LCA | Fucα6GlcNAc; High -Man | PHA_L | Tri/tetra-antennary complex-type N-glycan |
| GSL_II | Agalactosylated tri/tetra antennary glycans; GlcNAc | GSL_I_B 4 | αGal |
| UEA_I | Fucα2Galβ4GlcNAc (H-type 2) | DBA | Blood group A antigen; GalNAcα1-3GalNAc |
| LTL | Fucα3(Galb4)GlcNAc (Lex); Fucα2Galβ4GlcNAc (H-type 2) | WFA | GalNAcβ1-4GlcNAc; Galβ3(-6)GalNAc |
| MAL_I | Siaα2-3Galβ1-4GlcNAc | VVA | α-GalNAc; GalNAcα1-3Gal |
| TJA_I | Siaα2-6Gal/GalNAc | | |

On the basis of Table 1, the "step of determining the level of at least one glycan in a sample taken from the subject, the at least one glycan being at least one glycan that binds to at least one lectin selected from the group consisting of ACA, MAH, ABA, STL, LEL, WGA, MPA, Jacalin, MAL_I, PNA, ACG, GSL_I_A4, ConA, SSA, AOL, and GSL_II" in the section [1-1] can be rephrased as follows: "the step of determining the level of at least one glycan in a sample taken from the subject, the at least one glycan being selected from the group consisting of:
(Galβ1-4GlcNAc)ₙ;
(GlcNAcβ1-4)ₙ,;
(GlcNAcβ4MurNAc)ₙ;
Agalactosylated tri/tetra antennary glycans;
Fucα1-2Galβ1-4GlcNAc;
Fucα1-6GlcNAc;
GalNAc;
Galβ1-3GalNAc;
GlcNAc;
High-Man including Manα1-6(Manα1-3)Man;
Siaα2-3Galβ1-3(Siaα2-6)GalNAc;
Siaα2-3Galβ1-4GlcNAc;
Siaα2-6Gal/GalNAc; and
α-GalNAc".

Similarly, on the basis of Table 1, the "step of determining the level of at least one glycan in a sample taken from the subject, the at least one glycan being at least one glycan that binds to at least one lectin selected from the group consisting of ACA, MAH, ABA, MPA, Jacalin, LEL, ACG, STL, GSL_I_A4, WGA, SSA, PNA, ConA, Calsepa, AOL, SNA, UDA, LCA, GSL_II, UEA_I, LTL, MAL_I, TJA_I, ECA, PWM, PSA, AAL, DSA, BPL, TJA_II, NPA, PHA_E, RCA120, SBA, GNA, HHL, PTL_I, TxLC_I, PHA_L, DBA, WFA, and VVA" in the section [1-2] can be rephrased as follows: "the step of determining the level of at least one glycan in a sample taken from the subject, the at least one glycan being selected from the group consisting of:
(Galβ1-4GlcNAc)ₙ;
(GlcNAcβ1-4)ₙ;
(GlcNAcβ4)ₙ;
(GlcNAcβ4MurNAc)ₙ;
Agalactosylated tri/tetra antennary glycans;
bi- and tri-antennary N-glycans;
Blood group A antigen;
Fucα1-2Galβ1-4GlcNAc;
Fucα1-6GlcNAc;
Fucα2(Galα3)Galβ1-4GlcNAc;
Fucα2Galβ1;
Fucα2Ga1β4GlcNAc (H-type 2);
Fucα3(Galb4)GlcNAc (Lex);
Fucα3(Galβ4)GlcNAc (Lex);
Fucα6GlcNAc (core Fuc);
Fucα6GlcNAc;
GalNAc;
GalNAcα1-3Gal;
GalNAcα1-3GalNAc;
GalNAcβ1;
GalNAcβ1-4GlcNAc;
Gala1-3Galβ1-4GlcNAc;
Galβ1-3GalNAc;
Galβ1-4GlcNAc;
Galβ3(-6)GalNAc;
Galβ4GlcNAc;
GlcNAc;
GlcNAcβ4GlcNAc;
High-Man;
High-Man (Man2-6);
High-Man including Manα1-6(Manα1-3)Man;
High-Man including Manα3Man;
High-Man including Manα6Man;
Man3 core;
Man5 to Man9;
N-glycans including bisecting GlcNAc;
N-glycans with outer Gal and bisecting GlcNAc;
Siaα2-3Galβ1-3(Siaα2-6)GalNAc;
Siaα2-3Galβ1-4GlcNAc;
Siaα2-6Gal/GalNAc;
tetraantennary N-glycans;
Tri/tetra-antennary complex-type N-glycans;
triantennary;
αGal; and
α-GalNAc".

Furthermore, on the basis of Table 1, the "step of determining the level of at least one glycan in a first sample taken from the subject, the at least one glycan being at least one glycan that binds to at least one lectin selected from the group consisting of ACA, MAH, ABA, MPA, Jacalin, LEL, ACG, STL, GSL_I_A4, WGA, SSA, PNA, ConA, Calsepa, AOL, SNA, UDA, LCA, GSL_II, UEA_I, LTL, MAL_I, TJA_I, ECA, PWM, PSA, AAL, DSA, BPL, TJA_II, NPA, PHA_E, RCA120, EEL, SBA, HPA, GNA, HHL, PTL_I, TxLC_I, PHA_L, GSL_I_B4, DBA, WFA, and VVA" in each of the sections [2-1] and [2-2] can be rephrased as follows: "the step of determining the level of at least one glycan in a sample taken from the subject, the at least one glycan being selected from the group consisting of:
(Galβ1-4GlcNAc)ₙ;
(GlcNAcβ1-4)ₙ;
(GlcNAcβ4)ₙ;
(GlcNAcβ4MurNAc)ₙ;
Agalactosylated tri/tetra antennary glycans;
bi- and tri-antennary N-glycans;
Blood group A antigen;
Fucα1-2Galβ1-4GlcNAc;
Fucα1-6GlcNAc;
Fucα2Galβ1;
Fucα2Galβ4GlcNAc (H-type 2);
Fucα3(Galb4)GlcNAc (Lex);
Fucα6GlcNAc (core Fuc);
Fucα6GlcNAc;
GalNAc;
GalNAcα1-3Gal;
GalNAcα1-3GalNAc;
GalNAcβ1;
GalNAcβ1-4GlcNAc;
Galβ1-3GalNAc;
Galβ1-4GlcNAc;
Galβ3(-6)GalNAc;
Galβ4GlcNAc;
GlcNAc;
GlcNAcβ4GlcNAc;
High-Man;
High-Man(Man2-6) ;
High-Man including Manα1-6(Manα1-3)Man;
High-Man including Manα3Man;
High-Man including Manα6Man;
Man3 core;
Man5 to Man9;
N-glycans including bisecting GlcNAc;
N-glycans with outer Gal and bisecting GlcNAc;
Siaα2-3Galβ1-3(Siaα2-6)GalNAc;
Siaα2-3Galβ1-4GlcNAc;
Siaα2-6Gal/GalNAc;
tetraantennary N-glycans;
Tri/tetra-antennary complex-type N-glycans;
triantennary; and
α-GalNAc".

Furthermore, on the basis of Table 1, in each of the sections [2-1] and [2-2],
(1) "glycans that bind to the lectins ACA, GSL_I_A4, and GSL_II, respectively" can be rephrased as "(i) Galβ1-3GalNAc, (ii) α-GalNAc, and (iii) Agalactosylated tri/tetra antennary glycans or GlcNAc";
(2) "glycans that bind to the lectins MAH, GSL_I_A4, and MAL_I, respectively" can be rephrased as "Siaα2-3Galβ1-3(Siaα2-6)GalNAc, α-GalNAc, and Siaα2-3Galβ1-4GlcNAc";
(3) "glycans that bind to the lectins ABA, GSL_I_A4, and AOL, respectively" can be rephrased as "(i) Galβ1-3GalNAc, (ii) α-GalNAc, and (iii) Fucα1-6GlcNAc or Fucα1-2Galβ1-4GlcNAc";
(4) "glycans that bind to the lectins MAH, GSL_I_A4, and PNA, respectively" can be rephrased as Siaα2-3Galβ1-3(Siaα2-6)GalNAc, α-GalNAc, and Galβ1-3GalNAc;
(5) "glycans that bind to the lectins MAH, GSL_I_A4, and SNA, respectively" can be rephrased as "Siaα2-3Galβ1-3(Siaα2-6)GalNAc, α-GalNAc, and Siaα2-6Gal/GalNAc";
(6) "glycans that bind to the lectins ABA, GSL_I_A4, and MAL_I, respectively" can be rephrased as "Galβ1-3GalNAc, α-GalNAc, and Siaα2-3Galβ1-4GlcNAc";
(7) "glycans that bind to the lectins ABA, GSL_I_A4, and GSL_II, respectively" can be rephrased as "(i) Galβ1-3GalNAc, (ii) α-GalNAc, and (iii) Agalactosylated tri/tetra antennary glycans or GlcNAc"; and
(8) "glycans that bind to the lectins MAH, GSL_I_A4, and HPA, respectively" can be rephrased as "Siaα2-3Galβ1-3(Siaα2-6)GalNAc and α-GalNAc".

### (Presumed biological mechanism)

In Examples (described later), AUC was greatest for a diagnostic model in which glycans that bind to the lectins ACA, GSL_I_A4, and GSL_II, respectively were used in addition to three clinical factors. The combination of these glycans is, as described earlier, the combination of (i) Galβ1-3GalNAc, (ii) α-GalNAc, and (iii) Agalactosylated tri/tetra antennary glycans or GlcNAc.

This fact agrees with a known fact concerning IgA nephropathy. Specifically, it has been reported that insufficient galactosylation of O-glycan of IgA molecule is found in IgA nephropathy. In this regard, Galβ1-3GalNAc is a galactosylated O-glycan structure. Therefore, it is appropriate to interpret a decrease in level of Galβ1-3GalNAc as being related to IgA nephropathy.

Furthermore, it is inferred from the above fact that, because there is an increase in α-2,3-sialyltransferase activity in IgA nephropathy, α-GalNAc has been converted to Siaα2-3GalNAc and the level of α-GalNAc has decreased. Furthermore, agalactosylated tri/tetra-antennary glycans are N-glycan structures lacking galactose; this suggests that also galactosylation of N-glycan structure is insufficient in IgA nephropathy.

Note, however, that the above description is about a presumed mechanism to help understand the present invention, and is not intended to limit the scope of the present invention.

### [4. Kit]

### [4-1. Lectins included in kit]

A kit in accordance with an embodiment of the present invention includes lectins that bind to specific glycans contained in a sample taken from a subject. The descriptions in the sections [1] to [3] shall be referenced concerning such glycans and lectins that bind to the glycans.

A kit in accordance with an embodiment of the present invention is (i) a kit for determining the possibility that a subject has developed IgA nephropathy or (ii) a kit for determining the possibility that a subject having a primary glomerular disease or a subject suspected of having a primary glomerular disease has developed IgA nephropathy, the kit including at least one lectin selected from the following group A, at least one lectin selected from the following group B, and at least one lectin selected from the following group C, and not including other lectins:
Group A: ACA, MAH, and ABA;
Group B: GSL_I_A4; and
Group C: GSL_II, MAL_I, AOL, PNA, SNA, and HPA.

It is noted here that at least one lectin is selected from each of the groups A to C. Specifically, one lectin, two lectins, or three lectins is/are selected from the group A. One lectin is selected from the group B. One lectin, two lectins, three lectins, four lectins, five lectins, or six lectins is/are selected from the group C.

In an embodiment, lectins selected from the groups A to C are not the following combination: (i) ACA and/or ABA is/are selected from the group A, (ii) GSL_I_A4 is selected from the group B, and (iii) PNA and/or HPA is/are selected from the group C.

In an embodiment of the present invention, lectins selected from the groups A to C are not the following combination: (i) ACA and/or ABA is/are selected from the group A, (ii) GSL_I_A4 is selected from the group B, and (iii) PNA, SNA, and/or HPA is/are selected from the group C.

A kit in accordance with another embodiment of the present invention is (i) a kit for determining the possibility that a subject has developed IgA nephropathy or (ii) a kit for determining the possibility that a subject having a primary glomerular disease or a subject suspected of having a primary glomerular disease has developed IgA nephropathy, the kit including lectins that bind to at least one glycan selected from the following group a, at least one glycan selected from the following group b, and at least one glycan selected from the following group c, and not including other lectins:
Group a: Galβ1-3GalNAc and Siaα2-3Galβ1-3(Siaα2-6)GalNAc;
Group b: α-GalNAc;
Group c: Agalactosylated tri/tetra antennary glycans, Fucα1-2Galβ1-4GlcNAc, Fucα1-6GlcNAc, GlcNac, Galβ1-3GalNAc, Siaα2-3Galβ1-4GlcNAc, Siaα2-6Gal/GalNAc, and α-GalNAc.

It is noted here that at least one glycan is selected from each of the groups a to c. Specifically, one glycan or two glycans is/are selected from the group a. One glycan is selected from the group b. One glycan, two glycans, three glycans, four glycans, five glycans, six glycans, seven glycans, or eight glycans is/are selected from the group c.

Note that the number of lectins that bind to each glycan may be one or two or more. For example, in cases where an agalactosylated tri- or tetra- antennary glycan is selected from the group c, the kit in accordance with an embodiment of the present invention may include only one lectin that binds to the agalactosylated tri- or tetra- antennary glycan or may include two or more lectins that bind to the agalactosylated tri- or tetra- antennary glycan.

The same glycans may be selected from two or more groups. For example, α-GalNAc may be selected from each of the groups b and c. In such a case, the kit in accordance with an embodiment of the present invention may include only one lectin that binds to α-GalNAc or may include two or more lectins that bind to α-GalNAc.

In an embodiment, glycans selected from the groups a to c are not the following combination: (i) Galβ1-3GalNAc is selected from the group a, (ii) α-GalNAc is selected from the group b, and (iii) Galβ1-3GalNAc, Siaα2-6Gal/GalNAc, and/or α-GalNAc is/are selected from the group c.

In an embodiment, the lectins that bind to glycans selected from the groups a to c are not the combination of at least one selected from the following group 1 and at least one selected from the following group 2:
Group 1: BPL, ABA, Jacalin, PNA, ACA, and MPA
Group 2: HPA, VVA, PTL_I, and GSL_I_A4

The use of a kit that includes any of the earlier-described combinations of lectins makes it possible to determine the possibility that a subject has developed IgA nephropathy, with higher accuracy than conventional techniques. The subject may be a subject that belongs to an unspecified population or a subject having a primary glomerular disease or suspected of having a primary glomerular disease.

The forgoing lectins can be prepared by known methods. Alternatively, commercial lectins may be used as appropriate.

### [4-2. Other constituents]

In the kit in accordance with an embodiment of the present invention, lectins may be immobilized on a substrate. For example, lectins may be immobilized on a substrate such as a microarray, an ELISA plate, latex beads, magnetic beads, or the like.

Out of those listed above, an aspect in which the lectins are immobilized on a microarray is preferred. Such an aspect brings about the following advantages: in cases where urine is used as a sample, (i) the sample does not need to be concentrated and (ii) major proteins (such as albumin and IgG) do not need to be removed from the sample. The lectins can be immobilized on the substrate by a known method that involves immobilizing a protein on a substrate.

The kit in accordance with an embodiment of the present invention may further include agent(s), instrument(s), vessel(s), an instruction manual, and/or the like which are necessary in using the kit. The following configuration may be employed: a user obtains the agent(s), instrument(s), vessel(s), instruction manual, and/or the like from the market or via a communication line or the like.

### [5. Other aspects]

A method of determining the possibility that a subject has developed IgA nephropathy, in accordance with the present invention, is not a method of diagnosing IgA nephropathy carried out by a medical doctor, but instead a method to assist diagnosing IgA nephropathy in a subject.

Note, however, that the method of determining the possibility that a subject has developed IgA nephropathy in accordance with the present invention can be applied to a method of diagnosing IgA nephropathy. Thus, the present invention includes a "method of diagnosing IgA nephropathy" within its scope. Note that the descriptions concerning the "method of determining the possibility that a subject has developed IgA nephropathy" in the present specification can be employed as descriptions for the "method of diagnosing IgA nephropathy". In such a case, the term "method of determining the possibility that a subject has developed IgA nephropathy" can be read as "method of diagnosing IgA nephropathy".

The descriptions in the foregoing sections can apply as appropriate to other sections. The present invention is not limited to the embodiments, but can be altered variously within the scope of the claims. An embodiment derived from a combination of technical means each disclosed in a different embodiment is also encompassed in the technical scope of the present invention.

All academic and patent documents cited in the present specification are incorporated herein by reference.

The following description will discuss the present invention with reference to Examples. Note, however, that the present invention is not limited to these Examples.

### Examples

The following were studied with use of a method in accordance with an embodiment of the present invention: (i) the accuracy of diagnosis differentiating IgA nephropathy patient(s) from a population including healthy subjects and (ii) the accuracy of diagnosis differentiating between patient(s) having a primary glomerular disease and IgA nephropathy patient(s).

### [Method]

### [Selection of patients]

506 people were subjected to the analysis, which are part of 525 people including 510 kidney disease patients and 15 heathy subjects and which exclude 19 patients having both IgA nephropathy and another kidney disease. Note that prior consent was obtained from all the participants through a predetermined procedure. All the chronic kidney disease patients were those who underwent renal biopsy and received a confirmed diagnosis of a kidney disease at Okayama University Hospital from December 2010 to September 2017.

### [Measurement of glycan level via lectin array analysis]

Lectin arrays (GlycoStation [registered trademark, which will be omitted hereafter] and LecChip [registered trademark, which will be omitted hereafter] manufactured by GlycoTechnica Ltd.) were used to convert, into numerical form, the signal intensities of urinary glycans which bind to 45 lectins, in accordance with the following protocol. Urine samples used in the measurement are those which were taken prior to the renal biopsy or those which were taken for a medical checkup and which had been preserved.
1. 20 µL of a 10-fold diluted urine sample and Cy3 Mono-Reactive dye 100µg labeling (manufactured by GE Healthcare Life Science) were mixed and allowed to react for 1 hour at room temperature in a dark place.
2. A desalting column was subjected to centrifugation under the conditions of 1,500 xg, 1 minute, and 4°C.
3. Washing was carried out in the following manner: 300 µL of TBS was applied to the desalting column; and then the desalting column was subjected to centrifugation under the conditions of 1,500 xg, 1 minute, and 4°C. This step 3 was carried out three times.
4. The entire urine sample and 25 µL of TBS were applied to the desalting column. Then, centrifugation was carried out under the conditions of 1,500 xg, 2 minutes, and 4°C, thereby removing unreacted Cy3.
5. 450 µL of Probing Solution (manufactured by GlycoTechnica) was added to the urine sample obtained in the step 4, and then 500 µL was weight out.
6. LecChip was washed with Probing Solution three times (100 mL/well for each time). Then, the urine sample solution prepared in the step 5 was injected into the wells (100 µL/well).
7. The LecChip was allowed to react at 20°C for 16 hours or more.
8. The LecChip was subjected to measurement using GlycoStation Reader 1200. The measurement was carried out under the conditions in which the LecChip contains liquid like when the urine sample was undergoing a reaction. The total number of times the measurement was carried out was four, exposure time was 299 milliseconds, and camera gain was 85, 95, 105, 115, 125.
9. The measurement results were converted into numerical form using GlycoStaion ToolsPro Suite 1.5.
10. The value obtained by subtracting a background signal intensity from the signal intensity of each lectin was defined as a glycan signal of that lectin, and used for the analysis.

### [Clinical factor]

The following were measured as clinical factors: age at the time of renal biopsy, gender, BMI, blood pressure, serum Cr, serum IgA, complement C3, presence or absence of diabetic complication, HbAlc (NGSP), presence or absence of occult hematuria, and 24-hour urinary protein (g/day).

Of those listed above, the presence or absence of occult hematuria was determined in accordance with the Guideline for hematuria diagnosis 2013 (Japanese Society of Laboratory Medicine). Specifically, urine sediment analysis was carried out a plurality of times in early mornings before renal biopsy. In this analysis, if (i) the red blood cell count in urine was 5/HPF or more in two or more tests and (ii) it was determined from the form of red blood cells in urine that the blood was glomerular urinary blood, then it was determined as "occult hematuria".

eGFR (ml/min/1.73m²) was calculated from the value of serum Cr using CKD-EPI equation

The intensity of each glycan signal was multiplied by 1/1000, multiplied by 1/10000, or logarithmically converted depending on its distribution and intensity, and then subjected to the analysis. Similarly, the 24-hour urinary protein was converted to a natural logarithm and then subjected to the analysis.

### [Endpoint]

A differential diagnosis between subjects having IgA nephropathy and subjects not having IgA nephropathy was set as a primary endpoint (note, however, that cases having both IgA nephropathy and another kidney disease were excluded). Specifically, the following diagnosis was set as a primary endpoint: diagnosis to determine whether a subject is (i) a subject only having IgA nephropathy or (ii) a subject having a kidney disease other than IgA nephropathy or a healthy subject.

A differential diagnosis between subjects having IgA nephropathy and subjects having a primary glomerular disease was set as a secondary endpoint. Specifically, the following diagnosis was set as a secondary endpoint: diagnosis to determine whether a subject having a primary glomerular disease is (i) a subject only having IgA nephropathy or (ii) a subject having a primary glomerular disease other than IgA nephropathy.

### [Analysis]

A univariate logistic regression analysis or a multivariate logistic regression analysis was used to compare ROC-AUCs of the following four types of models and determine usefulness.
- Model (1): ROC-AUC of a multivariate logistic regression model composed of serum IgA (315 mg/dL or more vs. less than 315 mg/dL), presence or absence of occult hematuria, and 24-hour urinary protein (0.3 g/day or more vs. less than 0.3 g/day). Note that this model corresponds to a conventional technique.
- Model (2): ROC-AUC of a univariate logistic regression model for each of 45 lectin signals.
- Model (3): ROC-AUC of a composite multivariate logistic regression model composed of one lectin signal and three clinical factors in Model (1).
- Model (4): ROC-AUC of a composite multivariate logistic regression model composed of a plurality of lectin signals and three clinical factors in Model (1).

With regard to Model (1), "IgA nephropathy guidelines 2017" in Japan also proposes a diagnostic model in which serum IgA/complement C3 (>3.01 vs. <3.01) is used in addition to the above-mentioned three clinical factors, as a predictive model for diagnosis. However, in the populations used in this study, serum IgA and serum IgA/complement C3 were correlated very strongly. In view of this, serum IgA/complement C3 was excluded from the variates of Model (1) in consideration of the issue of multicollinearity. Note that the above-mentioned guideline also indicates that serum IgA is more important than serum IgA/complement C3, and it is considered that serum IgA more reflects the condition of IgA nephropathy. It is therefore inferred that Model (1), which is a clinical factor model, also has a certain degree of validity.

With regard to Model (3) and Model (4), serum IgA and 24-hour urinary protein were treated as continuous variables, not categorical variables with cutoff values. This is because there have been no evidence or report as to whether the conventionally-used cutoff values (see Model (1)) are generalizable also to Japanese IgA nephropathy patients.

With regard to Model (4), when a model including a plurality of lectin signals is to be prepared, the correlation between each lectin signal and clinical factors was studied, and those which had no strong correlation with the clinical factors were selected and entered. This was to avoid the issue of multicollinearity. Factors to be finally entered into the model were decided by a forward-backward stepwise method. The level of P-values was 0.1 in the forward-backward stepwise method.

With regard to models which were finally considered useful, sensitivity, specificity, and concordance were calculated using Youden Index and Optimum Distance. The analyses discussed in this section were all carried out with use of SAS (version 9.3, 9.4).

### [Results]

### [Result 1: Distribution of analyzed subjects]

Tables 2 and 3 each show distribution of analyzed subjects according to the type of kidney disease. Table 2 shows distribution of all analyzed subjects, and Table 3 shows distribution of all patients having a primary glomerular disease other than IgA nephropathy.

**[Table 2]**

| Table 2: Distribution of 506 analyzed subjects according to the type of kidney disease, and proportion of healthy subject | |
|---|---|
| Name of kidney disease (or healthy subject) | Number of cases (%) |
| IgA nephropathy | 157 (31) |
| Purpura nephritis (HSP) | 17 (3) |
| Vasculitis other than IgA nephropathy and HSP | 46 (9) |
| (including) ANCA-associated vasculitis | 34 (7) |
| Lupus nephritis | 36 (7) |
| Minimal change nephrotic syndrome | 29 (6) |
| Focal segmental glomerulosclerosis | 19 (4) |
| Membranous nephropathy | 31 (6) |
| Membranoproliferative glomerulonephritis | 12 (2) |
| Diabetic nephropathy | 25 (5) |
| Nephrosclerosis (including) Hypertensive nephrosclerosis | 24 (5) 21 (4) |
| Obesity-related nephropathy | 10 (2) |
| Interstitial nephritis/Acute tubular necrosis | 19 (4) |

**[Table 2] (Continued)**

| | |
|---|---|
| Thin basement membrane nephropathy | 10 (2) |
| Alport syndrome | 2 (0) |
| Cases having some other kidney disease or cases having two or more kidney diseases other than IgA nephropathy | 54 (11) |
| Healthy subject | 15 (3) |

Each number represents the number of cases having only one disease.

Note, however, that the analyzed subjects include cases with two or more diseases other than IgA nephropathy.

**[Table 3]**

| Table 3: Distribution and proportions of subjects having primary glomerular disease | |
|---|---|
| Primary glomerular disease (N=89) | Number of cases (%) |
| Minimal change nephrotic syndrome | 29 (33) |
| Focal segmental glomerulosclerosis (primary focal segmental glomerulosclerosis only) | 11 (12) |
| Membranous nephropathy | 31 (35) |
| Membranoproliferative glomerulonephritis | 12 (13) |
| Mesangial proliferative nephritis which is not IgA nephropathy | 2 (2) |
| Renal limited vasculitis | 4 (4) |

Each number represents the number of cases having only one disease.

Table 2 shows that, with regard to the primary endpoint, out of the 506 analyzed subjects, the number of cases only having IgA nephropathy was 157 (31%), and the number of cases not having IgA nephropathy was 349 (69%).

Table 3 shows that, with regard to the secondary endpoint, out of the 246 analyzed patients, the number of cases only having IgA nephropathy was 157 (31%), and the number of cases with a primary glomerular disease other than IgA nephropathy was 89 (36%). Out of the latter cases, the number of cases with membranous nephropathy was greatest (31), and the number of cases with minimal change nephrotic syndrome was second greatest (29).

Table 4 shows major clinical factors at the time of renal biopsy.

**[Table 4]**

| Table 4: Major clinical factors at the time of renal biopsy | | | | |
|---|---|---|---|---|
| Major clinical factors | All patients (n=506) | Subjects having IgA nephropathy (n=157) | Subjects not having IgA nephropathy (n=349) | *P*-Value |
| Male (%) | 51 | 49 | 51 | 0.52 |
| Age (in years) | 51 ± 18 | 42 ± 16 | 55 ± 17 | <0.001 |
| BMI (kg/m²) | 22.6 ± 3.8 | 22.2 ± 3.1 | 22.8 ± 4.0 | 0.19 |
| Systolic blood pressure (mmHg) | 126.8 ± 20.0 | 124.3 ± 17.6 | 128.0 ± 20.9 | 0.14 |
| Diastolic blood pressure (mmHg) | 78.5 ± 12.2 | 78.2 ± 12.3 | 78.6 ± 12.2 | 0.64 |
| Mean arterial pressure (mmHg) | 94.6 ± 13.5 | 93.6 ± 13.0 | 95.1 ± 13.7 | 0.25 |

**[Table 4] (Continued)**

| | | | | |
|---|---|---|---|---|
| High blood pressure** (%) | 44 | 31 | 50 | <0.001 |
| eGFR (ml/min/1.7 3m²) | 61.9 ± 28.9 | 70.5 ± 26.4 | 58.0 ± 29.2 | <0.001 |
| Amount of urinary protein (g/day)* | 0.92 (0.35 - 3.00) | 0.73 (0.27 - 1.53) | 1.23 (0.42 - 3.91) | <0.001 |
| Occult hematuria (%) | 62 | 85 | 52 | <0.001 |
| Serum IgA (mg/dl) | 283.6 ± 155.5 | 322.9 ± 198.8 | 265.1 ± 126.4 | 0.31 |
| Serum IgA ≥ 315 (mg/dl, %) | 34 | 44 | 30 | 0.002 |
| Complement C3 (mg/dl) | 99.9 ± 27.9 | 99.3 ± 17.7 | 100.1 ± 31.6 | 0.09 |

**[Table 4] (Continued)**

| | | | | |
|---|---|---|---|---|
| IgA/C3 ≥ 3.01 (%) | 42 | 51 | 38 | 0.005 |

| | | | | |
|---|---|---|---|---|
| BMI stands for body mass index. * Median (interquartile range) ** (i) Patients taking at least one hypotensive drug and (ii) patients having a systolic blood pressure of > 140 (baseline) or a diastolic blood pressure of >90 (baseline) were defined as having "high blood pressure". | | | | |

P-value is the significance probability for a comparison between the IgA nephropathy group (n=157) and the group not having IgA nephropathy (n=349).

The x² test was used to compare categorical variables, and the t-test or Mann-Whitney test was used to compare continuous variables depending on the distribution of the variables.

Table 4 shows that the proportion of males to all the analyzed subjects was 51%, the average age at the time of renal biopsy was 51, and the average BMI was 22.6 (kg/m²). Table 4 also shows that the average systolic blood pressure, the average diastolic blood pressure, and the average mean arterial pressure were 126.8 mmHg, 78.5 mmHg, and 94.6 mmHg, respectively, and the proportion of subjects with high blood pressure was 44%. With regard to clinical factors relating to renal function, the average eGFR was 61.9 ml/min/1,73m², the median of 24-hour urinary protein was 0.92 (g/day, quartile: 0.35 to 3.00), 62% of the analyzed subjects were positive for occult hematuria, and the average serum IgA was 283.6 mg/dL.

A comparison between the clinical factors of the IgA nephropathy group and the group not having IgA nephropathy shows that the IgA nephropathy group was younger, had a lesser proportion of high blood pressure patients, had higher eGFR, had a lesser amount of urinary protein, had a higher proportion of patients positive for occult hematuria, and had higher serum IgA, statistically significantly. These results affirm the validity of the conventional IgA nephropathy predictive model using serum IgA, presence or absence of occult hematuria, and 24-hour urinary protein.

### [Result 2: Differentiation of IgA nephropathy patients from unspecified population]

Table 5 shows the results of analyses of Model (2) and Model (3) with regard to the primary endpoint. Note that the AUC of Model (1) with regard to the primary endpoint was 0.617. Specifically, in differentiating patients only having IgA nephropathy from a population also including healthy subjects, the AUC of a diagnostic method corresponding to a conventional technique was 0.617.

**[Table 5]**

| Table 5: Results of logistic regression analysis of "one glycan" models and "one glycan + clinical factors" models | | |
|---|---|---|
| Name of Lectin | AUC ("one glycan" model) | AUC ("one glycan + clinical factors" model) |
| ACA | 0.691* | 0.801 |
| MAH | 0.674* | 0.797 |
| ABA | 0.665* | 0.797 |
| MPA | 0.650* | 0.783 |
| Jacalin | 0.641* | 0.780 |
| LEL | 0.658* | 0.778 |
| ACG | 0.628* | 0.776 |
| STL | 0.662* | 0.775 |
| GSL_I_A4 | 0.627* | 0.775 |
| WGA | 0.655* | 0.773 |
| SSA | 0.620* | 0.772 |
| PNA | 0.637* | 0.771 |
| ConA | 0.625* | 0.771 |
| Calsepa | 0.609 | 0.770 |
| AOL | 0.620* | 0.769 |
| SNA | 0.607 | 0.769 |
| UDA | 0.607 | 0.769 |
| LCA | 0.597 | 0.769 |
| GSL_II | 0.620* | 0.768 |
| UEA_I | 0.614 | 0.768 |
| LTL | 0.603 | 0.768 |
| MAL_I | 0.638* | 0.767 |
| TJA_I | 0.594 | 0.767 |
| ECA | 0.594 | 0.767 |
| PWM | 0.613 | 0.766 |
| PSA | 0.592 | 0.766 |
| AAL | 0.607 | 0.765 |
| DSA | 0.594 | 0.765 |
| BPL | 0.603 | 0.764 |
| TJA_II | 0.599 | 0.763 |
| NPA | 0.589 | 0.763 |
| PHA_E | 0.582 | 0.763 |
| RCA120 | 0.573 | 0.763 |
| EEL | 0.559 | 0.763 |
| SBA | 0.542 | 0.763 |
| HPA | 0.609 | 0.762 |
| GNA | 0.598 | 0.762 |
| HHL | 0.582 | 0.762 |
| PTL_I | 0.577 | 0.762 |
| TxLC_I | 0.568 | 0.762 |
| PHA_L | 0.547 | 0.762 |
| GSL_I_B4 | 0.546 | 0.762 |
| DBA | 0.546 | 0.762 |
| WFA | 0.539 | 0.762 |
| VVA | 0.590 | 0.761 |

| | | |
|---|---|---|
| *: "One glycan" model which showed higher AUC than that (0.617) of clinical factor model | | |

Table 5 shows that, in cases of Model (2), with regard to 16 lectins, the AUC of the model including that lectin signal was more than 0.617. Specifically, the following models showed an AUC greater than that of the conventional technique: models including the signal of a glycan that binds to ACA, MAH, ABA, STL, LEL, WGA, MPA, Jacalin, MAL_I, PNA, ACG, GSL_I_A4, ConA, SSA, AOL, or GSL_II. Out of these, the model that showed the greatest AUC was one including the signal of a glycan that binds to ACA. Next, the models including the signal of a glycan that binds to MAH, ABA, or STL showed an AUC of more than 0.660. Next, the models including the signal of a glycan that binds to LEL, WGA, MPA, or Jacalin showed an AUC of more than 0.640.

Table 5 also shows that, in cases of Model (3), with regard to all the 45 lectins, the models including the lectin signal showed an AUC of more than 0.617. That is, with regard to all the 45 lectins used in the analysis, an improvement of the conventional diagnostic method was achieved. Out of those mentioned above, the model that showed the greatest AUC was the model in which the signal of a glycan that binds to ACA was used in combination with clinical factors (0.801). The models in which the signal of a glycan that binds to ACA, MAH, or ABA was used in combination with clinical factors showed an AUC of more than 0.790. Next, the models in which the signal of a glycan that binds to MPA, Jacalin, LEL, ACG, STL, GSL_I_A4, WGA, SSA, PNA, or ConA was used in combination with clinical factors showed an AUC of more than 0.790 (note that, in Table 5, the lectins are arranged in descending order in terms of AUC of Model (3)).

Table 6 shows the results of analysis of Model (4) with regard to the primary endpoint.

**[Table 6]**

| Table 6: AUC and diagnostic accuracy for "two or more glycans + clinical factors" models | | | | | | | | |
|---|---|---|---|---|---|---|---|---|
| | "Two or more glycans + clinical factors" model | | | | | | | Clinical factors only |
| AUC | 0.807 | 0.806 | 0.806 | 0.805 | 0.805 | 0.805 | 0.803 | 0.617 |
| | ACA GSL_I_A4 GSL_II | MAH GSL_I_A4 MAL_I | ABA GSL_I_A4 AOL | MAH GSL_I_A4 PNA | MAH GSL_I_A4 SNA | ABA GSL_I_A4 MAL_I | ABA GSL_I_A4 GSL_II | |
| Sensitivity (Youden Index) | 0.764 | 0.739 | 0.752 | 0.771 | 0.752 | 0.777 | 0.732 | 0.840 |
| Specificity (Youden Index) | 0.754 | 0.784 | 0.763 | 0.757 | 0.787 | 0.731 | 0.760 | 0.364 |
| Concordance (Youden Index) | 0.758 | 0.770 | 0.760 | 0.762 | 0.776 | 0.745 | 0.752 | 0.516 |
| Sensitivity (Optimum Distance) | 0.764 | 0.739 | 0.752 | 0.771 | 0.752 | 0.777 | 0.732 | 0.474 |
| Specificity (Optimum Distance) | 0.754 | 0.784 | 0.763 | 0.757 | 0.787 | 0.731 | 0.760 | 0.675 |
| Concordance (Optimum Distance) | 0.758 | 0.770 | 0.760 | 0.762 | 0.776 | 0.745 | 0.752 | 0.611 |

Model (4) was analyzed, and it was found that the following seven types of models showed an AUC greater than the greatest AUC (0.801) of Model (3):
ACA+GSL_I_A4+GSL_II;
MAH+GSL_I_A4+MAL_I;
ABA+GSL_I_A4+AOL;
MAH+GSL_I_A4+PNA;
MAH+GSL_I_A4+SNA;
ABA+GSL_I_A4+MAL_I; and
ABA+GSL_I_A4+GSL_II.

Out of those mentioned above, the model which showed the greatest AUC was the model in which glycan models that bind to ACA, GSL_I_A4, and GSL_II were used in combination with clinical factors, and the AUC was 0.807. For this model, sensitivity was 0.764, specificity was 0.754, and concordance was 0.758. These results were the same between when Youden Index was used and when Optimum Distance was used.

In the above seven combinations, with regard to the lectin signals of ACA, MAH, ABA, and GSL_I_A4, low signal intensity was related to the development of IgA nephropathy. On the contrary, with regard to the lectin signals of GSL_II, MAL_I, AOL, PNA, and SNA, high signal intensity was related to the development of IgA nephropathy.

### [Result 3: Differentiation of IgA nephropathy patients from primary glomerular disease patients]

Table 7 shows the results of analyses of Model (2) and Model (3) with regard to the secondary endpoint. Note that the AUC of the Model (1) with regard to the secondary endpoint was 0.620. Specifically, in differentiating patients only having IgA nephropathy from a population of subjects having a primary glomerular disease, the AUC of a diagnostic method corresponding to the conventional technique was 0.620.

**[Table 7]**

| Table 7: Results of logistic regression analysis of "one glycan" models and "one glycan + clinical factors" models | | |
|---|---|---|
| Name of lectin | AUC ("one glycan" model) | AUC ("one glycan + clinical factors") |
| ABA | 0.677* | 0.884 |
| GSL_I_A4 | 0.668* | 0.881 |
| MAH | 0.686* | 0.880 |
| BPL | 0.714* | 0.879 |
| GSL_II | 0.697* | 0.879 |
| TJA_I | 0.685* | 0.879 |
| ConA | 0.731* | 0.878 |
| ACA | 0.703* | 0.878 |
| SSA | 0.697* | 0.878 |
| Calsepa | 0.706* | 0.877 |
| PNA | 0.673* | 0.877 |
| AOL | 0.711* | 0.876 |
| HPA | 0.618 | 0.876 |
| UDA | 0.691* | 0.875 |
| SNA | 0.688* | 0.875 |
| MPA | 0.672* | 0.875 |
| Jacalin | 0.671* | 0.875 |
| DBA | 0.631* | 0.875 |
| PWM | 0.713* | 0.874 |
| MAL_I | 0.713* | 0.874 |
| LEL | 0.693* | 0.874 |
| ECA | 0.676* | 0.874 |
| WFA | 0.664* | 0.874 |
| UEA_I | 0.662* | 0.874 |
| PTL_I | 0.656* | 0.874 |
| VVA | 0.651* | 0.874 |
| SBA | 0.648* | 0.874 |
| LTL | 0.641* | 0.874 |
| ACG | 0.641* | 0.874 |
| PHA_L | 0.628* | 0.874 |
| DSA | 0.685* | 0.873 |
| TJA_II | 0.675* | 0.873 |
| EEL | 0.606 | 0.873 |
| GSL_I_B4 | 0.581 | 0.873 |
| WGA | 0.711* | 0.872 |
| PHA_E | 0.680* | 0.872 |
| HHL | 0.687* | 0.871 |
| LCA | 0.670* | 0.871 |
| AAL | 0.707* | 0.870 |
| RCA120 | 0.668* | 0.870 |
| TxLC_I | 0.667* | 0.870 |
| GNA | 0.698* | 0.869 |
| NPA | 0.685* | 0.869 |
| PSA | 0.668* | 0.869 |
| STL | 0.679* | 0.868 |

| | | |
|---|---|---|
| *: "One glycan" model which showed higher AUC than that (0.620) of clinical factor model | | |

Table 7 shows that, in cases of Model (2), with regard to 42 lectins, the models including the lectin signal showed an AUC of more than 0.617. Specifically, the models including the signal of a glycan that binds to a lectin other than HPA, EEL, and GSL_I_B4 showed an AUC greater than that of the conventional technique. Out of these, the model that showed the greatest AUC was the model including the signal of a glycan that binds to ConA.

Table 7 also shows that, in cases of Model (3), with regard to all the 45 lectins, the models including the lectin signal showed an AUC of more than 0.620. That is, with regard to all the 45 lectins used in the analysis, an improvement of the conventional diagnostic method was achieved. Out of those mentioned above, the model that showed the greatest AUC was the model in which the signal of a glycan that binds to ABA was used in combination with clinical factors (0.884). The models in which any of the 30 lectin signals (signal of glycan that binds to ABA to signal of glycan that binds to PHA_L) in Table 7 was used in combination with clinical factors showed an AUC of more than 0.873. (Note that, in Table 7, the lectins are arranged in descending order in terms of AUC of Model (3).)

Table 8 shows the results of analysis of Model (4) with regard to the secondary endpoint.

**[Table 8]**

| Table 8: AUC and diagnostic accuracy for "two or more glycans + clinical factors" model | | |
|---|---|---|
| | "Two or more glycans + clinical factors" model | Clinical factors only |
| AUC | 0.899 | 0.620 |
| | MAH GSL_I_A4 HPA | |
| Sensitivity (Youden Index) | 0.860 | 0.840 |
| Specificity (Youden Index) | 0.841 | 0.364 |
| Concordance (Youden Index) | 0.853 | 0.668 |
| Sensitivity (Optimum Distance) | 0.860 | 0.474 |
| Specificity (Optimum Distance) | 0.841 | 0.682 |
| Concordance (Optimum Distance) | 0.853 | 0.549 |

Model (4) was analyzed, and it was found that the model that showed an AUC greater than the greatest AUC (0.884) of Model (3) was the model in which glycan models that bind to MAH, GSL_I_A4, and HPA were used in combination with clinical factors, and the AUC was 0.889. For this model, sensitivity was 0.860, specificity was 0.841, and concordance was 0.853. These results were the same between when Youden Index was used and when Optimum Distance was used.

In the above combination, with regard to the lectin signals of MAH and GSL_I_A4, low signal intensity was related to the development of IgA nephropathy. On the contrary, with regard to the lectin signal of HPA, high signal intensity was related to the development of IgA nephropathy.

### Industrial Applicability

The present invention can be used for, for example, diagnosis of IgA nephropathy.

## Claims

1. A method of determining the possibility that a subject has developed IgA nephropathy, the method comprising the step of:
determining a level of at least one glycan in a sample taken from the subject, the at least one glycan being at least one glycan that binds to at least one lectin selected from the group consisting of ACA, MAH, ABA, STL, LEL, WGA, MPA, Jacalin, MAL_I, PNA, ACG, GSL_I_A4, ConA, SSA, AOL, and GSL_II.

2. A method of determining the possibility that a subject has developed IgA nephropathy, the method comprising the steps of:
determining a level of at least one glycan in a first sample taken from the subject, the at least one glycan being at least one glycan that binds to at least one lectin selected from the group consisting of ACA, MAH, ABA, MPA, Jacalin, LEL, ACG, STL, GSL_I_A4, WGA, SSA, PNA, ConA, Calsepa, AOL, SNA, UDA, LCA, GSL_II, UEA_I, LTL, MAL_I, TJA_I, ECA, PWM, PSA, AAL, DSA, BPL, TJA_II, NPA, PHA_E, RCA120, EEL, SBA, HPA, GNA, HHL, PTL_I, TxLC_I, PHA_L, GSL_I_B4, DBA, WFA, and VVA; and
measuring a biomarker in a second sample taken from the subject, the biomarker being other than the level of the at least one glycan.

3. A method of determining the possibility that a subject having a primary glomerular disease or a subject suspected of having a primary glomerular disease has developed IgA nephropathy, the method comprising the step of:
determining a level of at least one glycan in a sample taken from the subject, the at least one glycan being at least one glycan that binds to at least one lectin selected from the group consisting of ACA, MAH, ABA, MPA, Jacalin, LEL, ACG, STL, GSL_I_A4, WGA, SSA, PNA, ConA, Calsepa, AOL, SNA, UDA, LCA, GSL_II, UEA_I, LTL, MAL_I, TJA_I, ECA, PWM, PSA, AAL, DSA, BPL, TJA_II, NPA, PHA_E, RCA120, SBA, GNA, HHL, PTL_I, TxLC_I, PHA_L, DBA, WFA, and VVA.

4. A method of determining the possibility that a subject having a primary glomerular disease or a subject suspected of having a primary glomerular disease has developed IgA nephropathy, the method comprising the steps of:
determining a level of at least one glycan in a first sample taken from the subject, the at least one glycan being at least one glycan that binds to at least one lectin selected from the group consisting of ACA, MAH, ABA, MPA, Jacalin, LEL, ACG, STL, GSL_I_A4, WGA, SSA, PNA, ConA, Calsepa, AOL, SNA, UDA, LCA, GSL_II, UEA_I, LTL, MAL_I, TJA_I, ECA, PWM, PSA, AAL, DSA, BPL, TJA_II, NPA, PHA_E, RCA120, EEL, SBA, HPA, GNA, HHL, PTL_I, TxLC_I, PHA_L, GSL_I_B4, DBA, WFA, and VVA; and
measuring a biomarker in a second sample taken from the subject, the biomarker being other than the level of the at least one glycan.

5. (i) A method of determining the possibility that a subject has developed IgA nephropathy or (ii) a method of determining the possibility that a subject having a primary glomerular disease or a subject suspected of having a primary glomerular disease has developed IgA nephropathy,
the method comprising the step of determining a level of at least one glycan in a sample taken from the subject, the at least one glycan being at least one selected from the group consisting of:
(Galβ1-4GlcNAc)ₙ;
(GlcNAcβ1-4)ₙ;
(GlcNAcβ4MurNAc)ₙ;
Agalactosylated tri/tetra antennary glycans;
Fucα1-2Galβ1-4GlcNAc;
Fucα1-6GlcNAc;
GalNAc;
Galβ1-3GalNAc;
GlcNAc;
High-Man including Manα1-6(Manα1-3)Man;
Siaα2-3Galβ1-3(Siaα2-6)GalNAc;
Siaα2-3Galβ1-4GlcNAc;
Siaα2-6Gal/GalNAc; and
α-GalNAc.

6. The method as set forth in claim 2 or 4, wherein the biomarker other than the level of the at least one glycan is at least one selected from occult hematuria, proteinuria, serum IgA, and serum IgA/C3 ratio.

7. The method as set forth in any one of claims 1 to 6, wherein the sample used in the step of determining the level of the at least one glycan is a urine sample.

8. (i) A kit for determining the possibility that a subject has developed IgA nephropathy or (ii) a kit for determining the possibility that a subject having a primary glomerular disease or a subject suspected of having a primary glomerular disease has developed IgA nephropathy,
the kit comprising at least one lectin selected from the following group A, at least one lectin selected from the following group B, and at least one lectin selected from the following group C, and not comprising other lectins:
Group A: ACA, MAH, and ABA;
Group B: GSL_I_A4; and
Group C: GSL_II, MAL_I, AOL, PNA, SNA, and HPA.

9. (i) A kit for determining the possibility that a subject has developed IgA nephropathy or (ii) a kit for determining the possibility that a subject having a primary glomerular disease or a subject suspected of having a primary glomerular disease has developed IgA nephropathy,
the kit comprising lectins that bind to at least one glycan selected from the following group a, at least one glycan selected from the following group b, and at least one glycan selected from the following group c, and not comprising other lectins:
Group a: Galβ1-3GalNAc and Siaα2-3Galβ1-3(Siaα2-6)GalNAc;
Group b: α-GalNAc;
Group c: Agalactosylated tri/tetra antennary glycans, Fucα1-2Galβ1-4GlcNAc, Fucα1-6GlcNAc, GlcNac, Galβ1-3GalNAc, Siaα2-3Galβ1-4GlcNAc, Siaα2-6Gal/GalNAc, and α-GalNAc.
